# EUROPEAN PATENT SPECIFICATION

(11) **EP 3 836 877 B1**
(45) Date of publication and mention of the grant of the patent: **08.01.2025**
(21) Application number: 19869704.7
(22) Date of filing: 14.05.2019
(51) Int. Cl.: A61F 13/56, A61F 13/00, A61F 13/62

(54) **A SOFT AND BREATHABLE NONWOVEN-BASED FRONTAL CONNECTION ELEMENT WITH INCREASED HOOK-AND-LOOP FASTENING PERFORMANCE IN BABY AND ADULT DIAPERS AND THE PRODUCTION METHOD OF SAID FRONTAL CONNECTION ELEMENT**
WEICHES UND ATMUNGSAKTIVES VORDERES VERBINDUNGSELEMENT AUF VLIESSTOFFBASIS MIT VERBESSERTER KLETTVERSCHLUSSLEISTUNG IN BABY- UND ERWACHSENENWINDELN UND VERFAHREN ZUR HERSTELLUNG DES VORDEREN VERBINDUNGSELEMENTS
ÉLÉMENT DE CONNEXION AVANT À BASE DE NON-TISSÉ SOUPLE ET PERMÉABLE À L'AIR AYANT UNE PERFORMANCE DE FIXATION VELCRO ACCRUE DANS DES COUCHES POUR BÉBÉS ET ADULTES ET PROCÉDÉ DE PRODUCTION DUDIT ÉLÉMENT DE CONNEXION AVANT

(30) Priority: 16.08.2018 TR 201812018
(43) Date of publication of application: 23.06.2021
(73) Proprietor: Sareks Ambalaj Sanayi Ve Ticaret A.S., Tekirdag (TR)
(72) Inventor: IPEKLER, Serhat, Istanbul (TR)
(74) Representative: Kaya, Erdem
(86) International application number: PCT/TR2019/050328
(87) International publication number: WO 2020/072014

(56) References cited:
- EP-A1- 1 290 960
- WO-A1-99/14045
- US-A1- 2003 176 846
- US-A1- 2011 015 608
- US-A1- 2011 152 819
- US-A1- 2014 154 459
- US-B2- 9 125 775

## Description

### TECHNICAL FIELD

The present invention relates to a disposable diaper embodiment like baby diaper and patient diaper.

### PRIOR ART

Disposable diapers like baby diapers, sanitary pads and patient diapers are among the requirements of our daily life because of need and usage easiness. Particularly in baby diapers, said diapers shall not be accidentially opened through the bonding regions due to the mobility of babies and small children, body temperature, etc. and this is as important as the absorbability of said diaper.

During use, the flexible male connection part is fixed to the front part of the diaper and the diaper is fastened to the user. Male and female connection parts are used in open/close structure. Hook-like protrusions provided on the male connection part engage onto the female connection elements provided at the front part and which are in nonwoven structure. The front part, which has nonwoven structure, is embodied so as to have a structure where the layers are bonded so as to be formed by different layers or in a stand-alone manner, and the front part shall not be delaminated during the opening and closing process, in other words, the layers forming the structure shall not separate/delaminate from each other easily as a result of the effect of moving/forcing of opening/closing of the layers which form the structure. At the same time, the front part shall have at least partially flexible structure for providing opening/closing easiness. Thus, the binding proportion and shape/pattern of the layers, forming the front part which is in bonded structure, gain importance.

One of the works in the relevant technical field is patented in publication US9125775 B2. This invention relates a mechanical fastening system used in various products, particularly absorbent articles like training pants, diapers, and incontinence garments. These articles typically have a fastening system that allows them to be refastened for user convenience.

The common hook-and-loop system used in these applications has certain drawbacks, including hook detachment during active movements, which can lead to inconvenience and leakage. To overcome these issues, this invention introduces a hook-and-loop fastening system with a unique nonwoven loop laminate material. This material is bonded in a way that provides it with higher z-directional strength between its layers. It can be used in various products, including disposable training pants, diapers, and non-absorbent items. In a preferred embodiment, the mechanical fastening system includes a first component with the nonwoven loop laminate material and a second component with a hook material, both attached to the article. The nonwoven loop laminate material is made with post bonding, enhancing its durability and reducing the risk of detachment during use. The invention also covers methods of producing this post-bonded loop laminate material suitable for hook-and-loop fastening systems.

In the invention with publication number WO2010135508A1; a bonded region embodiment, which is in wavy form provided at the nonwoven front part, is described. Said bonded region is essentially embodied in a parallel manner to each other and multiple in number.

In the patent with publication number WO9914045A1; a diaper component is described which is made of a pre-bonded nonwoven layer and a film layer and having bonded and non-bonded regions thereon. The film used in said connection has a multi-layered structure and the parts which provide connection with the nonwoven comprise amorphous polymer. Said diaper is particularly baby diaper. Said embodiment is used on the outer surface of the baby diaper for holding of the male connection element. It is used for completely wrapping the outer surface.

In the patent with publication number EP1216679; a laminated fibrous material production method is described which describes connection of at least one fibrous net material having the first bonding pattern and at least one second fibrous net material having the second bonding pattern. Said first and second bonding patterns have bonded regions with different dimensions.

### BRIEF DESCRIPTION OF THE INVENTION

The present invention relates to a front part embodiment in disposable diapers, for eliminating the above mentioned disadvantages and for bringing new advantages to the related technical field.

An object of the present invention is to provide a front part embodiment where cost is reduced and where functionality is not compromised.

Another object of the present invention is to provide a front part embodiment having reduced % bonding area and having greater strength.

Another object of the present invention is to provide a front part embodiment which provides a comfortable/reliable open-close system by means of the male connection element.

Another object of the present invention is to provide a front part where the problem of pilling of the nonwoven, which forms the front part after the open-close movement/function of the male connection element through the front part for a few times, is reduced and where the open-close performance loss is reduced as much as possible.

In order to realize all of the abovementioned objects and the objects which are to be deducted from the detailed description below, the present invention is a front part with layered structure provided on a main body provided on a diaper and to which a male connection part is connected in an openable-closeable manner. Accordingly, said front part is characterized in that
- said front part comprises a first nonwoven layer which is between 18 gsm and 55 gsm in weight and which is the part whereon the male connection part is held,
- said first nonwoven layer is formed by means of at least one of spundbond, air through, meltblown, spunmelt and carded thermobonded type comprising pre-bonded staple fibers which has total percentage of pre-bonded area on carded thermobonded web preferably between 9% and 12%,
- a first nonwoven layer comprises at least one of polypropylene, polyethylene and polyethylene terephthalate;
- said front part comprises a second nonwoven layer which is between 8 gsm and 20 gsm in weight and which is positioned between the main body (20),
- said second nonwoven layer is formed by means of at least one of spunbond and spunmelt in terms of the production method,
- second nonwoven layer (33) comprises at least one of polypropylene, polyethylene and polyethylene terephthalate,
- pluralities of bonded regions having application density between 15% and 30% on the front part and which define the regions where the first nonwoven layer and the second nonwoven layer are connected by means of thermal bonding.

In a preferred embodiment of the present invention, each bonded region is in the form of at least one of island-form, star-like, short line, curved line, wavy line, shallow wavy line, shallow wavy line circular dots, curved, snow flake, elliptical form in a manner not intersecting with each other.

In another preferred embodiment of the present invention, the bonded regions are in the form of discontinuous dotted shallow wavy lines and make an angle between 10° and 18° with respect to axis X.

In another preferred embodiment of the present invention, the peel strength of the front part after the third opening of the male connection part is at least 3.5 N/25 mm.

In another preferred embodiment of the present invention, the bonded regions have embossing depth between 0.7 mm and 0.9 mm onto the nonwoven material provided in the bonding areas.

In another preferred embodiment of the present invention, in the bonded regions in curved/s-wave form, there is a distance between 2.5 mm and 4.0 mm between the two connection lines in parallel placement in direction MD.

In another preferred embodiment of the present invention, when the bonded regions are brought together, the bonded regions are in the form of engaged snowflakes when viewed from outside and make an angle between 6° and 10° with respect to axis X and make an angle between 8° and 11° with respect to axis Y.

### BRIEF DESCRIPTION OF THE FIGURES

Figure 1 is a representative laminated cross sectional view of the front part.
Figure 2 is a representative view of the shallow wavy embodiment of the front part.
Figure 3 is a representative view of the baby diaper.
Figure 4 is a representative view of the male connection part.
Figure 5 is a representative view of the curved wavy embodiment of the front part.
Figure 6 is a representative view of the elliptical - snow pattern embodiment of the front part.
Figure 7 is a representative view of another shallow wavy embodiment of the front part.

### REFERENCE NUMBERS

10 Diaper
20 Main body
30 Front part
   31 First nonwoven layer
   33 Second nonwoven layer
   34 Bonded region
   35 Non-bonded region
   36 Center
40 Lateral band
   41 Main carrier
   42 Male connection part
CD direction: →
MD direction: ↑

### DETAILED DESCRIPTION OF THE INVENTION

In this detailed description, the subject matter front part (30) of the disposable diaper (10) is explained with references to examples without forming any restrictive effect only in order to make the subject more understandable.

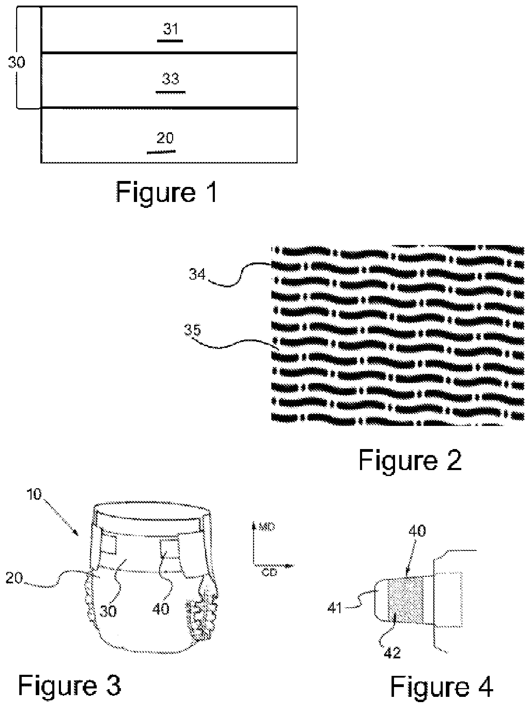

With reference to Figure 1, in order to form the diaper (10), there is a main body (20) and a lateral band (40) connected to said main body (20). When the diaper (10) is in open position, the direction which extends from the lateral band (40) towards the main body (20) is named as CD direction (→) and the direction from the lateral band (40) towards the length of the diaper (10) is named as the MD direction (↑). Said main body (20) comprises a front part (30). Said front part (30) essentially has textiled surface and/or loop touch structure made of textile (it is called nonwoven in the related art, and hereafter, it will be called nonwoven in the specification).

Said front part (30) has a layered structure and it comprises first nonwoven layer (31) and second nonwoven layer (33). There is at least one bonded region (34) which defines the regions where the first nonwoven layer (31) and the second nonwoven layer (33) are connected and there is at least one non-bonded region (35) which defines the regions outside said bonded region (34). There is at least one center (36) defined between the ends of the bonded regions (34) which are close to each other.

Said lateral band (40) made of at least one thermoplastic-based material essentially comprises a main carrier (41) and a male connection part (42) connected onto said main carrier (41).

The first nonwoven layer (31) and the second nonwoven layer (33) are preferably made of nonwoven material. Nonwoven is in reality a thermoplastic-based material. The nonwoven material used in the invention is preferably made of thermoplastic PP material. In alternative embodiments, PP/PE, PET, viscose and derivatives thereof can be used.

The first nonwoven layer (31) is the part where the male connection part (42) is held during use and consists of fibers based on thermoplastic material. The first nonwoven layer (31) can be structures where 100% PP (polypropylene) or PP is mixed/blended with various proportions of PE (polyethylene) or where PP is mixed with PET (polyethylene teraphatalate) or where PET and PE are mixed. The first nonwoven layer (31) preferably has PP structure, and depending on the nonwoven production method, it can be carded thermobonded comprising pre-bonded staple fibers or spunbond or air through bonded, meltblown, spunmelt or the mixture thereof. However, there has to be no meltblown or melt structure therein. In cases where the first nonwoven layer (31) has pre-bonded structure, the pre-bonding area percent is between 10% and 20%, in the preferred application, it is between 10% and 15%, in the further preferred application, it is between 10% and 12%. The weight of the first nonwoven layer (31) is between 18 gsm and 55 gsm, in the preferred application, it is between 18 gsm and 31 gsm, in the further preferred application, it is between 21 gsm and 31 gsm.

The second nonwoven layer (33) is positioned on the main body (20) and it consists of thermoplastic fibers. The first nonwoven layer (31) can be structures where 100% PP (polypropylene) or where PP is mixed with various proportions of PE (polyethylene) or where PP is mixed with PET (polyethylene teraphatalate) or where PET and PE are mixed at various proportions. The second nonwoven layer (33) can have bi-component fiber structure so as to comprise at least 40% PP or it can have mono-component fiber structure formed by homogenous material formed completely by a single material. It preferably may be in the form of 100% PP or mixture/blend in various proportions of PP, PE or PET. Depending on the nonwoven production method, the second nonwoven layer (33) can preferably consist of structures like spunbond, spunmelt, spunlace, air through, air laid or the combinations of the mixtures of them. The weight of the second nonwoven layer (33) is between 8 gsm and 20 gsm, in the preferred application, it is between 9 gsm and 16 gsm, and in the further preferred application, it is between 10.8 gsm and 13.2 gsm.

In the invention, the first nonwoven layer (31) and/or the second nonwoven layer (33) can be connected by means of at least one of the connection types of ultrasonic and/or thermal and/or adhesive through bonded regions (34). In the preferred application, thermal bonding method is used for providing the connection.

On the front part (30), the bonded regions (34) are positioned in an independent manner from each other and essentially they have a short structure and they have a form which repeats at a predetermined pattern. Each bonded region (34) is in the form of at least one of island-form, star-like, short line, curved line, wavy line, shallow wavy line, shallow wavy line circular dots, curved, snow flake, elliptical form in a manner not intersecting with each other.

The distance between the end part of a bonded region (34) and the end part of the adjacent bonded region (34) which extends in the same axis as said bonded region (34) is at least 1 mm and at most 10 mm. In the preferred application, the distance between the end part of the bonded region (34) and the end part of the adjacent bonded region (34) which repeats as the symmetric in the form of continuation of said bonded region (34) in the same axis as said bonded region (34) is at least 1.5 mm and at most 8 mm. In the further preferred application, the distance between the end part of the bonded region (34) and the end part of the adjacent bonded region (34), which repeats as the symmetric in the form of continuation of said bonded region (34) in the same axis as said bonded region (34), is at least 1.8 mm and at most 6 mm.

The depth of embossing onto the nonwoven material in the bonding areas of the bonded region (34) is between 0.5 mm and 1.0 mm, and in the preferred application, it is between 0.7 mm and 0.9 mm.

In an embodiment of the present invention, the bonded regions (34) have elliptical structure. The length of one bonded region (34) in this embodiment is between 2 mm and 10 mm. In the preferred application, the length of one bonded region (34) is between 3 mm and 4 mm. The length of one bonded region (34) is 2.45 mm. The width of one bonded region (34) is preferably at most 1.4 mm.

With reference to Figure 2, in an embodiment of the present invention, the bonded regions (34) are in the form of discontinuous dotted shallow wave form. In said embodiment, based on the bonded regions (34), the placement makes an angle between 10° and 18° with respect to the horizontal axis (X axis). The length of the bonded region which is in shallow wave form is between 16 mm and 22 mm and the width thereof is between 0.8 mm and 1.4 mm.

The positioning forms of the bonded regions (34) in direction MD are parallel with respect to each other, and the distances of sequential parallel bonded region (34) in direction MD are between 2.5 mm and 4.0 mm.

The length between two bonded regions (34) which are in dot form provided between both of the two bonded regions (34) positioned side by side and with shallow wave, in other words the length between the two dots in direction CD is between 20 mm and 26 mm. The diameter of bonded regions (34), which are in dot form, is between 0.9 mm and 1.4 mm.

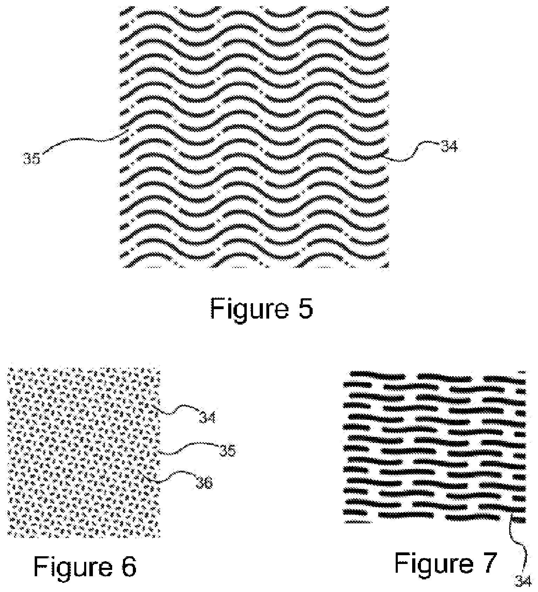

With reference to Figure 7, in an embodiment of the present invention, the bonded regions (34) are in the form of discontinuous shallow wave form. In this embodiment, the length of the non-bonded region provided between both of the two bonded regions (34) positioned side by side is between 3 mm and 7 mm in direction CD. The bonded region (34) width or unit length in MD direction is between 0.8 mm and 1.4 mm.

With reference to Figure 5, in an embodiment of the present invention, the bonded regions (34) are embodied in the form of deep wavy line with discontinuous dots.

In said embodiment, the width of the bonded region is between 0.8 mm and 1.4 mm. The distance between the two bonded regions (34) in the orthogonal axis (MD direction) is between 2.5 mm and 4.0 mm.

The length between the bonded regions (34) in dot form provided between the bonded regions (34) positioned side by side and which are in curved deep wave form, in other words, the length between the two points in CD direction is between 22 mm and 27 mm. The diameter of the bonded regions (34), which are in dot form, is between 0.8 mm and 1.4 mm.

In an embodiment of the present invention, the bonded regions (34) extend outwardly from said center (36) in one of the positioning types on the front part (30). Preferably, when the bonded regions (34) extend outwardly from the center (36) and when the ends of the bonded regions (34) are joined, a hexagonal appearance is formed. As the number of bonded regions (34) extending outwardly from the center (36) increases, a circular appearance is formed when the bonded region (34) ends are joined.

With reference to Figure 6, in an embodiment of the present invention, when the bonded regions (34) are brought together, they are positioned to create snow flakes apperance engaged into each other when viewed from outside. The bonded regions (34) are discontinuous and do not intersect and contact with each other. Another characteristic of the bonded regions (34) is that bonded regions (34) are not encircled by contour. There is a precalculated distance between at least two bonded regions (34). There is the non-bonded region (35) in this distance in between. The non-bonded regions (35) are defined essentially in the part which remains between at least two bonded regions (35). The non-bonded regions (35) are in continuous structure. In said embodiment, the bonded regions (34) make an angle of 6°-10° with respect to axis X and make an angle of 8°-11° with respect to axis Y. The width of the bonded regions (34) is between 0.8 mm and 1.4 mm, and the length of the bonded regions (34) is between 2.2 mm and 2.6 mm. The length of one bonded region (34) is preferably 2.45 mm.

The proportion of the area occupied by the bonded regions (34) at the front part (30) in the direction of MD (↑) and CD (→), in other words, the application density in the total is between 15% and 30%. In the preferred embodiments, the application density is between 15% and 27%. In a specific application, the application density of the bonded region (34) is 19.8%. In another embodiment, the application density of the bonded region (34) is between 23% and 27%.

The bonded region (34) provides a more resistant female-male connection thanks to the shape thereof and the proportion thereof in the total area, and the production efficiency (thermal lamination quality and speed) increases.

In the invention, thanks to a front part (30) structure which does not intersect with each other and which is more flat and where the bonded region (34) proportion is reduced, during opening and re-connecting of the male connection part (42) from the front part (30), the pilling of the front part (30) is prevented.

Particularly, during the following opening and closing processes, the functionality of the front part (30) is preserved since pilling is prevented. At the same time, thanks to its structure, a softer and more comfortable structure is obtained in terms of touch.

In an application of the present invention, circular and/or point pre-bonding process is applied to the first nonwoven layer (31). Afterwards, the first nonwoven layer (31) and the second nonwoven layer (33) are joined by using at least one of ultrasonic or thermal or glued lamination methods. Preferably, thermal bonding method is used for joining.

In another application of the present invention, the first nonwoven layer (31) has air through bonded structure. It comprises at least one of PP or PE or PET in its body at a specific proportion.

The second nonwoven layer (33), which is the lower layer, has varieties of thermoplastic material. It comprises at least one of specific amount of PP or PE in its body at a specific proportion.

While thermal binding method is being applied, at least one of snow flake, polygon-like, brick arrangement like, star-like, diagonal-like, pluralities of wave patterns, which are essentially parallel with respect to each other, can be applied to the front part (30) by bringing together bonded regions (34) which are in cut short line form. Each bonded region (34) can be in the form of a short straight line form which are independent from each other, or each bonded region (34) can be in the form of half "S" form or deep curved wavy or shallow wavy which are independent from each other. In different applications, similar forms and/or different pattern applications can also be used. The bonded regions (34) are positioned in an angled manner with respect to each other. In another embodiment, the bonded regions (34) can be positioned in a sequential manner with respect to each other.

Since the bonded regions (34) of the front part (30) have repetitive structures which are independent from each other within the mentioned dimensions, although the total binding proportion is reduced when compared with the present art, there is no reasonable decrease in the peeling strength value, and particularly in some applications, higher peel/shear strength values can be obtained. Thanks to this structure, opening and closing process, which is comfortable and safe, can be provided by means of the male connection part (42).

In said embodiments, pilling problem, which directly affects functionality of the front part (30), does not occur as a result of opening-closing of the male connection part (42) for a few times or stays at the minimal level and thus, the usage of front part (30) with low cost and comprising nonwoven structure is provided. While all of these are provided, a problem like delamination is not faced between the joined parts.

Moreover, while it has been claimed that desired performance can be get by using carded thermobonded nonwoven of non-pre-bonded type and addressing the upper layer nonwoven only to the carded thermobonded type class and having high percentage of total thermally bonded area (34) that is minimum 35% and maximum 55% (peel shear, lamination quality) in the present art,by subject matter embodiment, a much lower total thermally bonded area (34) is obtained by means of the subject matter embodiments, and equivalent performance is obtained by using pre-bonded type carded thermobonded nonwoven.

By means of this, without depending on a single supplier or production plant, the front part (30) can be produced by using carded thermobonded nonwoven products from different suppliers.

Moreover, the other differences of the subject matter embodiment are as follows;
- By using a thermal pre-bonded type nonwoven, there is no obligation to realize synchronization with an offline thermobonding production process or on a specific line with nonwoven production. This substantially decreases the required (cumulative) line investment cost for realizing such a type of production.
- The preference reason of nonwoven front part (30) is the soft touch, 3D moldable view, sufficient peel/shear strength performance and breathability.
   Regarding softness/ soft touch, is that, as higher total % bonded regions (34) exist, hardness feel will increase and softness in turn will be compromised since a crystallized structure will form partially or completely in the structure of the thermal bonded regions
   However, in the subject matter invention, functionality and soft touch are not compromised and an advantageous structure is provided when compared with the other products which exist in the art.
- The front part (30), which is one of the subject matter embodiments and which is in laminated structure belonging to the pattern whose detailed placement is shown in Figure 6, is much more resistant in MD/CD and particularly in crosswise and diagonal directions and particularly said front part (30) is more stable against the tensions in the baby diaper machine.
- The tension force is distributed into various directions in a more rapid and balanced manner when compared with the products which exist in the present art and particularly much more effective results can be obtained when compared with the embodiments which exist in the present art.
- Particularly by using more bulky, moldable and high-volume NWs like air through bonded type and which are not pre-bonded and which are not in the carded thermobonded class, the upper layer pilling problem is minimized thanks to the low total bonded region (34) proportion through much closer points.
- In a similar manner, by using nonwoven which is pre-bonded and which is in carded thermobonded class,
- the upper layer pilling or nonwoven linting problem by hook and loop interaction
- can be minimized thanks to much lower % bonded area and more frequent regional bonding.

For peeling - shearing tests (as 180 peel strength test and 180 shear strength test known in the art) of the products obtained by the subject matter embodiment, as male connection part, Gottlieb Binder 8564X and similar class hook type is used which provides good peeling and shearing performance. The lateral band male connection part (42) width taken as 13 mm and lateral band (40) cut length taken as 25 mm.

When the applications where the bonded region (34) application density is 15-27% are compared with the front part (30) products of the present art, the subject matter front part (30) shows 180 degrees peel strength which is greater than 10% for the sequential open-close action thanks to the bonding pattern and to the first nonwoven layer (31). This is a very important subject for usage functionality and comfort in baby and adult diapers.

**Table 1: Results of peeling strength for 180 degrees measured in increasing opening numbers**

| **180 peel N/25 mm (*) (Application load / Application area)** | **Peeling strength after 1^{st} opening (N)** | **Peeling strength after 2^{nd} opening (N)** | **Peeling strength after 3^{rd} opening (N)** |
|---|---|---|---|
| **Subject matter product** | **4.0** | **5.3** | **5.7** |
| **Product in the present art** | **5.3** | **3.3** | **3.2** |

In the subject matter embodiment, the front part (30) shows higher peel (180 peels) performance when compared with the present art as a result of the multiple open-close tests of the front part (30). In the products of the present art, the bonded region proportions are 28% and above.

By means of the subject matter designs, although the bonding proportion is at most 27%, the 180 degrees peeling strength stays almost the same when compared with the products of the present art and it is observed that said peeling strength even increases as a result of some tests. This is a very important technical gain.

The protection scope of the present invention is set forth in the annexed claims and cannot be restricted to the illustrative disclosures given above, under the detailed description. It is because a person skilled in the relevant art can obviously produce similar embodiments under the light of the foregoing disclosures, without departing from the main principles of the present invention.

## Claims

1. A front part (30) with layered structure provided for a diaper (10), said diaper (10) comprises a main body (20) and a lateral band (40) in connection with the main body (20), said front part (30) is on the main body (20) and is connected in an openable-closeable manner with a male connection part (42) included in the lateral band (40), the front part (30) has pluralities of reliable/comfortable open-close and 180 degrees peeling performance by means of the male connection part (42) and the peeling strength is increased by at least 10% when compared with the present art in terms of the 180 degrees peeling performance and in terms of the second and third open-close following the first open-close **characterized in that**
- said front part (30) comprises a first nonwoven layer (31) which is between 18 gsm and 55 gsm in weight and which is the part whereon the male connection part (42) is held,
- said first nonwoven layer (31) is formed by means of at least one of spunbond, air through, meltblown, spunmelt and carded thermobonded type comprising pre-bonded staple fibers which has total percentage of pre-bonded are on carded thermobonded web preferably between 9% and 12%,
- the first nonwoven layer (31) comprises at least one of polypropylene, polyethylene and polyethylene terephthalate,
- said front part (30) comprises a second nonwoven layer (33) which is between 8 gsm and 20 gsm in weight and which is positioned between the main body (20),
- said second nonwoven layer (33) is formed by means of at least one of spunbond and spunmelt in terms of the production method,
- the second nonwoven layer (33) comprises at least one of polypropylene, polyethylene and polyethylene terephthalate;
- pluralities of bonded regions (34) having application density between 15% and 30% on the front part (30) and which define the regions where the first nonwoven layer (31) and the second nonwoven layer (33) are connected by means of thermal bonding.

2. The front part (30) according to claim 1, **wherein** each bonded region (34) is in the form of at least one of island-form, star-like, short line, curved line, wavy line, shallow wavy line, shallow wavy line circular dots, curved, snow flake, elliptical form in a manner not intersecting with each other.

3. The front part (30) according to claim 2, **wherein** the bonded regions (34) are in the form of discontinuous dotted shallow wavy lines and make an angle between 10° and 18° with respect to axis X.

4. The front part (30) according to claim 1, **wherein** the bonded regions (34) have embossing depth between 0.7 mm and 0.9 mm onto the nonwoven material provided in the bonding areas.

5. The front part (30) according to claim 2, **wherein** in the bonded regions (34) in curved/s-wave form, there is a distance between 2.5 mm and 4.0 mm between the two connection lines in parallel placement in direction MD.

6. The front part (30) according to claim 2, **wherein** when the bonded regions (34) are brought together, the bonded regions (34) are in the form of engaged snow flakes when viewed from outside and make an angle between 6° and 10° with respect to axis X and make an angle between 8° and 11° with respect to axis Y.

## Patentansprüche

1. Vorderteil (30) mit geschichteter Struktur, das für eine Windel (10) vorgesehen ist, wobei die Windel (10) einen Hauptkörper (20) und ein seitliches Band (40) in Verbindung mit dem Hauptkörper (20) umfasst, wobei das Vorderteil (30) auf dem Hauptkörper (20) ist und in einer zu öffnenden und zu schließenden Weise mit einem männlichen Verbindungsteil (42) verbunden ist, das in dem seitlichen Band (40) enthalten ist, das Vorderteil (30) mittels des männlichen Verbindungsteils (42) mehrere zuverlässige/bequeme Öffnungs-/Schließvorgänge und 180-Grad-Schälvorgänge aufweist und die Schälfestigkeit im Vergleich zum gegenwärtigen Stand der Technik in Bezug auf die 180-Grad-Schälvorgänge und in Bezug auf den zweiten und dritten Öffnungs-/Schließvorgang, der auf den ersten Öffnungs-/Schließvorgang folgt, um mindestens 10% erhöht ist, **dadurch gekennzeichnet, dass**
- das Vorderteil (30) eine erste Vliesstoffschicht (31) mit einem Gewicht zwischen 18 g/m² und 55 g/m² umfasst, die der Teil ist, auf dem das männliche Verbindungsteil (42) gehalten wird,
- die erste Vliesstoffschicht (31) mittels mindestens einem der Typen Spinnvlies, Luftdurchsatz, Meltblown, Spunmelt und kardiertes Thermobonded gebildet wird, der vorgebundene Stapelfasern umfasst, deren Gesamtprozentsatz an vorgebundenen Fasern auf kardiertem Thermobonded-Vlies vorzugsweise zwischen 9% und 12% liegt,
- die erste Vliesstoffschicht (31) mindestens eines von Polypropylen, Polyethylen und Polyethylenterephthalat umfasst,
- der vordere Teil (30) eine zweite Vliesstoffschicht (33) mit einem Gewicht zwischen 8 g/m² und 20 g/m² umfasst, die zwischen dem Hauptkörper (20) angeordnet ist,
- die zweite Vliesstoffschicht (33) durch mindestens eines der Verfahren Spunbond und Spunmelt im Rahmen des Herstellungsverfahrens gebildet wird,
- die zweite Vliesstoffschicht (33) mindestens eines von Polypropylen, Polyethylen und Polyethylenterephthalat umfasst;
- mehrere verbundene Bereiche (34) eine Auftragsdichte zwischen 15% und 30% auf dem Vorderteil (30) aufweisen und die Bereiche definieren, in denen die erste Vliesstoffschicht (31) und die zweite Vliesstoffschicht (33) durch thermische Verfestigung verbunden sind.

2. Vorderteil (30) nach Anspruch 1, **wobei** jeder verbundene Bereich (34) die Form von mindestens einer der folgenden Formen aufweist: inselförmig, sternförmig, kurze Linie, gebogene Linie, Wellenlinie, flache Wellenlinie, flache Wellenlinie, kreisförmige Punkte, gebogen, Schneeflocke, elliptische Form in einer Weise, die sich nicht gegenseitig überschneidet.

3. Vorderteil (30) nach Anspruch 2, **wobei** die verbundenen Bereiche (34) die Form von unterbrochenen, gepunkteten, flachen Wellenlinien haben und mit der Achse X einen Winkel zwischen 10° und 18° bilden.

4. Vorderteil (30) nach Anspruch 1, **wobei** die verbundenen Bereiche (34) eine Prägetiefe zwischen 0,7 mm und 0,9 mm auf das in den Bindungsbereichen vorgesehene Vliesmaterial aufweisen.

5. Vorderteil (30) nach Anspruch 2, **wobei** in den verbundenen Bereichen (34) in Kurven-Is-Wellenform ein Abstand zwischen 2,5 mm und 4,0 mm zwischen den beiden Verbindungslinien in Parallelanordnung in Richtung MD besteht.

6. Vorderteil (30) nach Anspruch 2, **wobei,** wenn die verbundenen Bereiche (34) zusammengebracht werden, die verbundenen Bereiche (34) von außen betrachtet die Form von ineinandergreifenden Schneeflocken haben und einen Winkel zwischen 6° und 10° in Bezug auf die Achse X und einen Winkel zwischen 8° und 11° in Bezug auf die Achse Y bilden.

## Revendications

1. Une partie avant (30) avec une structure en couches est fournie pour une couche-culotte (10), ladite couche-culotte (10) comprend un corps principal (20) et une bande latérale (40) en liaison avec le corps principal (20), ladite partie avant (30) se trouve sur le corps principal (20) et est reliée de manière à pouvoir être ouverte et fermée à une partie de connexion mâle (42) incluse dans la bande latérale (40), la partie avant (30) présente plusieurs performances d'ouverture-fermeture fiables/confortables et de pelage à 180 degrés grâce à la partie de connexion mâle (42) et la force de pelage est augmentée d'au moins 10% par rapport à l'art actuel en termes de performance de pelage à 180 degrés et en termes de deuxième et troisième ouverture-fermeture suivant la première ouverture-fermeture, **caractérisée en ce que**
- ladite partie avant (30) comprend une première couche de non-tissé (31) dont le poids est compris entre 18 g/m² et 55 g/m² et qui est la partie sur laquelle la partie de connexion mâle (42) est maintenue,
- ladite première couche de non-tissé (31) est formée au moyen d'au moins l'un des filé-lié, air à travers, fusion-soufflage, filé-fondu et thermolié cardé, comprenant des fibres discontinues préliées dont le pourcentage total de fibres préliées sur la bande thermoliée cardée se situe de préférence entre 9% et 12%,
- la première couche non tissée (31) comprend au moins l'un des polypropylènes, polyéthylènes et polyéthylènes téréphtalates,
- ladite partie avant (30) comprend une deuxième couche de non-tissé (33) dont le poids est compris entre 8 g/m² et 20 g/m² et qui est placée entre le corps principal (20),
- ladite deuxième couche de non-tissé (33) est formée au moyen d'au moins l'un des filé-lié et filé-fondu, en fonction de la méthode de production,
- la deuxième couche non tissée (33) comprend au moins l'un des polypropylènes, polyéthylènes et polyéthylènes téréphtalates;
- des pluralités de régions liées (34) ayant une densité d'application comprise entre 15% et 30% sur la partie avant (30) et qui définissent les régions où la première couche de non-tissé (31) et la seconde couche de non-tissé (33) sont connectées au moyen d'une liaison thermique.

2. La partie avant (30) selon la revendication 1, **dans laquelle** chaque région liée (34) se présente sous la forme d'au moins une des île, étoile, ligne courte, ligne courbe, ligne ondulée, ligne ondulée peu profonde, ligne ondulée peu profonde, points circulaires, courbe, flocon de neige, forme elliptique de manière à ne pas se croiser les unes les autres.

3. Partie avant (30) selon la revendication 2, **dans laquelle** les régions liées (34) se présentent sous la forme de lignes discontinues pointillées peu profondes ondulées et forment un angle compris entre 10° et 18° par rapport à l'axe X.

4. La partie avant (30) selon la revendication 1, **dans laquelle** les régions liées (34) ont une profondeur de gaufrage comprise entre 0,7 mm et 0,9 mm sur le matériau non tissé fourni dans les zones de liaison.

5. La partie avant (30) selon la revendication 2, **dans laquelle** dans les régions liées (34) en forme de courbe/onde s, il y a une distance entre 2,5 mm et 4,0 mm entre les deux lignes de connexion en placement parallèle dans la direction MD.

6. La partie avant (30) selon la revendication 2, **dans laquelle** lorsque les régions liées (34) sont rapprochées, les régions liées (34) ont la forme de flocons de neige engagés lorsqu'elles sont vues de l'extérieur et forment un angle compris entre 6° et 10° par rapport à l'axe X et un angle compris entre 8° et 11° par rapport à l'axe Y.
